Europäisches Patentamt

European Patent Office

Office européen des brevets

(19)

(11) Numéro de publication: **0 069 659**
**B1**

(12) **FASCICULE DE BREVET EUROPEEN**

(45) Date de publication du fascicule du brevet:
09.04.86

(21) Numéro de dépôt: 82401236.3

(22) Date de dépôt: 01.07.82

(51) Int. Cl.⁴: **C 07 C 101/24,** C 07 C 129/12,
A 61 K 31/195, A 61 K 31/33,
A 61 K 35/74, A 61 K 45/02 //
(A61K45/02,
31:195),(A61K45/02, 31:195,
35:74),(A61K35/74,
31:195),(A61K31/33, 31:195)

(54) Médicament comprenant le produit de la réaction d'un acide carboxylique sur un amino-acide basique.

(30) Priorité: 03.07.81 FR 8113190

(43) Date de publication de la demande:
12.01.83 Bulletin 83/2

(45) Mention de la délivrance du brevet:
09.04.86 Bulletin 86/15

(84) Etats contractants désignés:
AT BE CH DE FR GB IT LI LU NL SE

(56) Documents cités:
FR - A - 1 012 750
US - A - 3 024 272

CHEMICAL ABSTRACTS, vol.65, no.3, 1er août 1966,
colonne 4477a, Columbus, Ohio (US), S. TOLNAI et al.:
"The in vitro antitumor activity of fatty acids. VII. Effect
of amino acid-fatty acid salts"
CHEMICAL ABSTRACTS, vol.92, no.3, 21 janvier 1980,
page 75, résumé no.15687c, Columbus, Ohio (US), S.
TANIGUCHI et al.: "Subacute toxicity of L-lysine acetate
in rats"
CHEMICAL ABSTRACTS, vol.92, no.13, 31 mars 1980,
page 692, résumé no.111290p, Columbus, Ohio (US)

(73) Titulaire: CHANY, Charles, 17/19 rue Emile Dubois,
F-75014 Paris (FR)

(72) Inventeur: Chany, Charles, 17-19, rue Emile Dubois,
F-75014 Paris (FR)
Inventeur: Cerutti, Italina, née Burolla, 119, rue des
Pyrénées, F-75020 Paris (FR)

(74) Mandataire: Koch, Gustave, Cabinet
PLASSERAUD 84, rue d'Amsterdam, F-75009 Paris (FR)

LIBER, STOCKHOLM 1986

## Description

L'invention a pour objet un médicament comprenant comme substance active le produit de la réaction — ci-après appelé "produit" — d'un acide carboxlique du groupe comprenant les acides butyrique, propionique et hexanoïque, sur un amino-acide basique du groupe constitué par l'arginine et la lysine; les produits ainsi obtenus sont appelés ci-après butyrate, propionate et hexanoate.

Elle vise également les médicaments comprenant le susdit produit et l'interféron.

Elle vise également les médicaments comprenant le susdit produit et un agent immunostimulant.

Elle vise enfin les médicaments comprenant le susdit produit, de l'interféron et un agent immunostimulant.

Parmi les susdits produits, le butyrate est particulièrement préféré.

Il est connu de l'art antérieur, notamment du brevet français FR-A-1 012750 (Merck), des produits de réaction d'amino-acides basiques (arginine, lysine) avec des acides carloscyliques inférieurs (acide acétique), comme solutionspoui injections parentérales bien tolérées.

En outre, il est aussi connu du chemical Abstracts vol. 65, No 3 (1er août 1966) réf. 4477a, S. Tolnai et al, comme antiturnoraux in vitro, des sels notamment de la lysine avec des acides gras.

Il est également, connu du Chemical Abstracts vol. 92 No 13, (13.3.1980) p. 692 ref. 111290 p, des sels de Ne-trimithyl-1-lysine avec les acides aspartique, funnarique ou glutamique, comme immunostimulants er pouraccroître l'effet et diminner la roscicité de produits cytostatiques.

De plus, le sel de sodium de l'acide butyrique a été décrit comme augmentant l'effet antitumoral et l'effet antiviral de l'interféron.

Administré seul, le butyrate de sodium ne présente aucun de ces deux effets.

Il était, par conséquent, impossible de prévoir que les butyrates, propionates et hexanoates des susdits amino-acides basiques, qui n'avaient jamais été utilisés jusqu'alors en médecine, auraient, appliqués seuls, un effet antiviral et antitumoral.

C'est pourtant ce que les Inventeurs ont eu le mérite de démontrer à l'issue de recherches approfondies.

Il s'ensuit que le médicament conforme à l'invention est caractérisé par le fait qu'il comprend, en tant que substance active, au moins l'un des produits de la réaction d'un acide carboxylique du groupe comprenant les acides butyrique, propionique et hexanoïque sur l'arginine ou la lysine, de préférence du butyrate d'arginine ou de lysine.

Les Inventeurs ont également montré que les produits en question et notamment lesdits butyrates d'aminoacides basiques stimulaient l'action antitumorale de l'interféron et des agents immunostimulants.

Il s'ensuit que le médicament conforme à l'invention est également caractérisé par le fait qu'il comprend, en tant qus substance active, au moins l'un des produits en question et, de préférence, du butyrate d'arginine ou de lysine ainsi que de l'interféron et/ou un agent immunostimulant.

Le médicament conforme à l'invention est également caractérisé par le fait qu'outre la substance active il comprend un excipient non toxique et pharmacologiquement acceptable.

Le médicament conforme à l'invention est administré par les voies parentérale, intraveineuse ou orale.

Il se présente avantageusement sous les formes pharmaceutiques constituées par les solutions isotoniques du produit (administrables par exemple par la voie intraveineuse) ou par des poudres ou ampoules buvables pour l'administration orale, comprenant le butyrate sous forme lyophilisée dans le cas des poudres et sous forme de solution aqueuse dans le cas des ampoules buvables. Dans le cas du butyrate d'arginine, la solution isotonique contient 178 millimoles de produit par litre de solution. Il est intéressant de noter que le médicament à base de butyrate ne présente pas l'odeur désagréable de l'acide butyrique.

Lorsque le médicament comprend également de l'interféron et/ou certains agents immunostimulants, la voie parentérale est possible. Dans ce cas, le médicament comprend les deux ou même les trois principes actifs éventuellement sous des conditionnements distincts. La possibilité d'avoir en présence l'un de l'autre notamment le butyrate et l'interféron existe également.

La quantité efficace
— de produit, de préférence de butyrate d'amino-acide basique et, éventuellement,
— d'interféron et/ou
— d'agent immunostimulant
est celle qui, administrée à la souris, provoque:
— un effet antiviral décelable par la survie de souris qui ont été infectées par 100 fois la $DL_{50}$ du virus de l'encéphalomyocardite en ce qui concerne le produit, notamment le butyrate d'amino-acide basique, pris seul (la présence d'interféron et/ou d'agent immunostimulant pouvant améliorer encore les résultats),
— un effet antitumoral décelable par l'augmentation de la survie moyenne de souris auxquelles on a greffé une tumeur ascitique, en ce qui concerne le produit, notamment le butyrate d'amino-acide basique, tant utilisé seul que conjointement à l'interféron et/ou à l'agent immunostimulant.

La dose pour un jour peut être, en ce qui concerne le produit, notamment le butyrate d'amino-acide basique, utilisé seul, comprise entre 0,28 mg et 0,56 mg par g de souris (ce qui correspond à la quantité administrée à une souris de 25 g par un demi-millilitre d'une solution contenant 50 millimoles par litre en ce qui concerne la limite inférieure et contenant 100 millimoles par litre en ce qui concerne la limite supérieure).

D'autres essais ont montré que cette dose pouvait être encore notablement abaissée, et ce jusqu'à une gamme d'environ 0,07 mg à 0,009 mg de produit par gramme de souris (ce qui correspond à l'administration à une souris de 25 g d'un demi-millilitre d'une solution à 12,5 mmoles par litre en ce qui concerne la limite supérieure et d'une solution de 1,6 mmoles par litre en ce qui concerne la limite inférieure).

Dans le cas de l'administration à l'homme, on peut également s'attendre à ce que la dose probable de 10 à 20 g de substance active par jour puisse être abaissée, notamment jusqu'à 0,5 g par jour.

Lorsqu'on utilise conjointement le produit, notamment le butyrate et l'interféron et/ou l'agent immunostimulant,
— la dose de produit, notamment de butyrate, est inchangée,
— la dose d'interféron administrable à l'homme est comprise entre 1 et $5.10^6$ unités d'interféron,
— la dose d'agent immunostimulant est fonction de la nature de celui-ci.

Par exemple, dans le cas du corynébactérium parvum pris en tant qu'agent immunostimulant, la dose unique du traitement (appliquée 18 heures avant la greffe cellulaire expérimentale, au moment de celle-ci ou trois jours plus tard) est de 200 microgrammes pour une souris de 25 g. Dans le cas de l'administration à l'homme, cette dose est celle généralement utilisée pour ce produit dans les applications déjà connues.

Le produit, notamment le butyrate d'amino-acide basique, est de préférence administré en plusieurs fois; il en est de même pour l'interféron.

La posologie est adaptée en fonction du sujet et de l'affection traitées.

L'agent immunostimulant peut être:
— le corynébacterium parvum,
— l'isoprinosine,
— l'extrait de staphylocoques,
— la cimétidine,
— le lévamisole.

Un médicament conforme à l'invention particulièrement préféré est à base:
— soit de produit de la réaction d'acide hexanoïque et, plus préférentiellement encore, d'acide butyrique sur l'arginine et comprend éventuellement de l'interféron et/ou un agent immunostimulant,
— soit de produit de la réaction d'acide hexanoïque et, plus préférentiellement encore, d'acide butyrique sur la lysine et comprend éventuellement de l'interféron et/ou un agent immunostimulant.

L'absence de toxicité du produit, notamment du butyrate d'amino-acide basique, aux doses susdites a été démontrée par des expériences effectuées sur la souris.

En effet, administré à la dose de 5 à 10 g/kg de poids du corps par jour à un groupe de 15 souris, on ne constate aucun décès au bout de 100 jours.

Les coefficients thérapeutiques de l'interféron et des agents immunostimulants, notamment ceux susmentionnés, sont bien connus et il n'y a pas lieu, par conséquent, d'y revenir ici.

L'excipient entrant dans la constitution du médicament conforme à l'invention peut être l'eau distillée ou encore une solution tamponnée comprenant éventuellement les agents conservateurs habituels.

La préparation des produits de la réaction de ces acides carboxyliques, notamment des butyrates d'amino-acides basiques entrant dans la constitution de la substance active des médicaments conformes à l'invention, peut être effectuée par neutralisation de l'acide, notamment de l'acide butyrique, par l'amino-acide basique jusqu'à pH 7, voire jusqu'à pH 7,3.

A titre d'exemple, on indique la préparation des butyrates d'arginine et de lysine.

Les deux butyrates en question sont préparés en ajoutant goutte l'amino-acide basique à l'acide butyrique et en suivant l'évolution du pH au pH-mètre. La neutralité est obtenue, c'est-à-dire que le butyrate se trouve préparé par addition d'une mole d'acide butyrique à une mole d'amino-acide basique. Le sel obtenu ne présente pas l'odeur désagréable de l'acide butyrique.

Les constantes physiques et/ou chimiques du butyrate d'arginine sont:
— Formule brute: $C_{10}H_{22}O_4N_4$, $H_2O$
— Poids moléculaire: 280,3
— Point de fusion: 145°C
— Pouvoir rotatoire: 16,6
— pH à 1 %: 7,3.

Les expériences qui ont permis aux Inventeurs de démontrer l'activité antivirale du produit et notamment du butyrate d'amino-acide basique pris seul et l'activité antitumorale de ce produit seul ou lorsqu'il est mis en oeuvre conjointement avec de l'interféron et/ou un agent immunostimulant, vont à présent être décrites.

Tout d'abord, on décrit les expériences montrant l'activité antivirale des butyrates d'amino-acides basiques pris seuls.

Dans ces expériences, on met en évidence l'activité antivirale contre l'effet léthal de 100 $DL_{50}$ du virus de l'encéphalomyocardite.

Les animaux de laboratoire utilisés sont des souris du type "Swiss" provenant d'un élevage propre aux Inventeurs.

Le virus employé est celui de l'encéphalomyocardite de la souris (EMC) de la souche Mengo cultivée sur des cellules murines L 929.

Le titre des suspensions de virus est estimé par la "méthode des plages" en utilisant toujours des cellules murines L 929. On admet que 5 unités (UFP) formant des plages, correspondent à une dose léthale $DL_{50}$.

Le virus est inoculé par voie intrapéritonéale.

Le processus expérimental consiste à inoculer à des groupes de 15 animaux:
— dans un premier temps, le butyrate d'arginine (0,28 mg par gramme de souris) à un groupe et le butyrate de lysine (0,24 mg par gramme de souris) à un autre groupe,
— dans un deuxième temps, au bout d'environ 18 heures, 100 $DL_{50}$ de virus.

On réalise également, à titre de comparaison, les expériences suivantes:
— prétraitement des animaux par le butyrate de sodium avant inoculation du virus EMC,

— traitement par le butyrate d'arginine ou de lysine stimulanément à l'injection du virus.

Des groupes de souris témoin sont traités par le virus seul et le solvant (milieu isotonique) seul.

En réalisant deux séries d'expériences avec des groupes de 15 souris, on constate que, au bout de 60 jours,

— dans les groupes témoins, 94 % (85 sur 90) des animaux sont morts,

— dans les groupes traités au butyrate de sodium, 90 % (81 sur 90) des animaux meurent; il n'est pas possible de parler de protection (le pourcentage de signification généralement dénommé p est supérieur à 95 %, étant rappelé que la définition de "p" peut être trouvée dans l'ouvrage de D. Schwarts, "Méthodes statistiques à l'usage des Médecins et des Biologistes", édité par Flammarion),

— dans les groupes traités au butyrate de lysine, 75 % (34 sur 45) des animaux meurent ($p < 0,01$),

— dans les groupes traités au butyrate d'arginine, 77 % (58 sur 75) des animaux meurent ($p < 0,01$),

— dans les groupes traités soit au butyrate d'arginine, soit au butyrate de lysine, simultanément à l'injection du virus, aucun effet protecteur n'est décelé.

On décrit ensuite les expériences montrant l'action antitumorale du butyrate d'amino-acide basique lorsqu'il est mis en oeuvre soit seul, soit parallèlement à l'interféron et/ou à un agent immunostimulant.

On souligne en effet que le butyrate d'amnio-acide basique, notamment d'arginine ou de lysine, présente une activité antitumorale significative lorsqu'il est mis en oeuvre seul, cette activité devenant plus importante lors de l'administration simultanée avec l'une au moins des deux autres substances actives susmentionnées.

La tumeur utilisée dans les expériences qui suivent est un mutant du sarcome de Crocker 180 TG qui résiste au traitement par les dérivés puriques.

Les animaux d'expériences sont toujours des souris Swiss de l'élevage susmentionné.

On leur inocule le sarcome par voie intrapéritonéale en utilisant une suspension de $10^6$ cellules/0,5 ml. La quantité inoculée est de 0,5 ml.

Les expériences ont été réalisées en utilisant:

— le butyrate d'arginine ou de lysine,

— l'interféron,

— le corynébacterium parvum (ou CP) de souche Mérieux.

Le butyrate d'arginine a été mis en oeuvre sous la forme et en quantité analogue à ce qui est indiqué plus haut pour l'activité antivirale.

Il en est de même pour le butyrate de lysine.

Le CP est administré dans les expériences qui suivent sous la forme d'une dose unique appliquée au moment de la greffe ou trois jours après et se présentant sous la forme d'ampoules de 2 ml comprenant 4 mg en poids sec de germes tués par le formol et la chaleur; on injecte 0,1 ml de cette substance à la souris par voie intrapéritonéale.

On a procédé à neuf expériences indépendantes portant chacune sur plusieurs groupes de 15 animaux.

Dans la première de ces expériences (groupe témoin), on a greffé aux animaux la tumeur seule.

Les animaux des autres groupes se sont également vu greffer la susdite tumeur; ils ont en plus été traités:

— soit par le butyrate d'arginine (deuxième expérience),

— soit par le butyrate de lysine (troisième expérience),

— soit par l'interféron (quatrième expérience),

— soit par le CP (cinquième expérience),

— soit par le butyrate d'arginine plus l'interféron (sixième expérience),

— soit par le butyrate de lysine plus l'interféron (septième expérience),

— soit par le butyrate d'arginine plus le CP (huitième expérience),

— soit par le butyrate d'arginine plus le CP plus l'interféron (neuvième expérience).

Dans les deuxième et troisième expériences, le butyrate d'amino-acide basique a été appliqué par voie intrapéritonéale à raison de 0,5 ml d'une solution 50 fois millimolaire par souris (c'est-à-dire à raison de respectivement 0,28 mg et 0,24 mg par g de souris), trois fois par semaine (à 48 heures d'intervalle) pendant trois semaines.

Dans la quatrième expérience, on a appliqué l'interféron à raison de 20.000 à 50.000 U.I. sous un volume de 0,5 ml administré à l'animal trois fois par semaine (à 48 heures d'intervalle) pendant trois semaines.

Dans la cinquième expérience, on a appliqué le CP en une dose unique de 200 microgrammes /0,1 ml par animal, administrée par voie intrapéritonéale en même temps ou trois jours après la greffe cellulaire.

Dans les sixième à neuvième expériences, on a appliqué, suivant le cas, le butyrate d'amino-acide basique, l'interféron et/ou le CP aux mêmes doses, au même rythme et aux mêmes moments que lorsque ces produits étaient appliqués seuls dans les expériences précédentes.

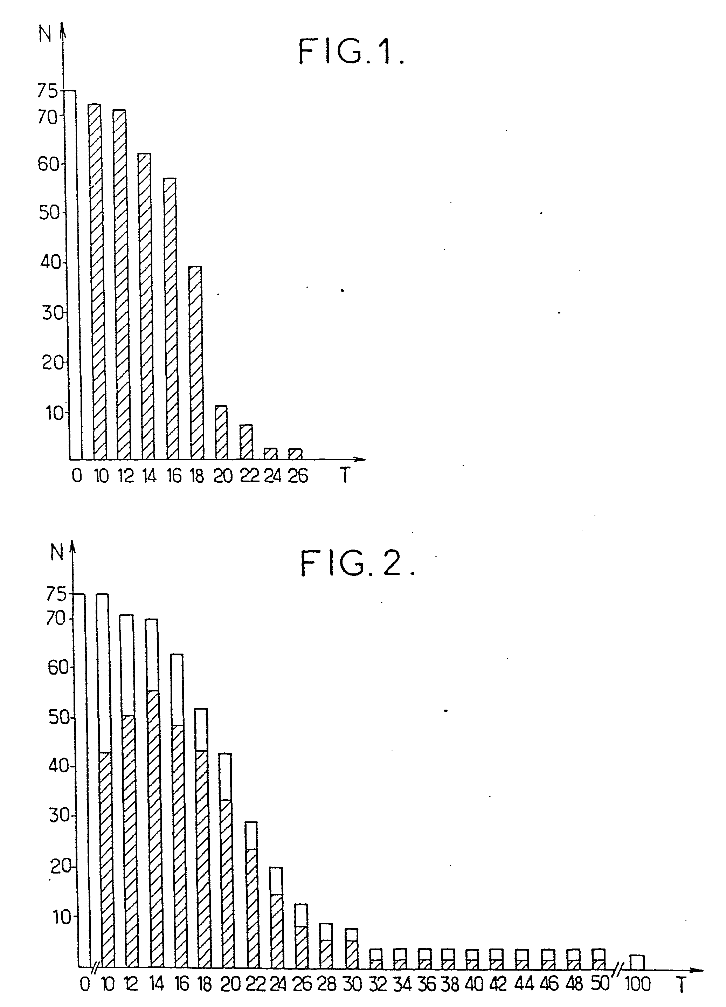

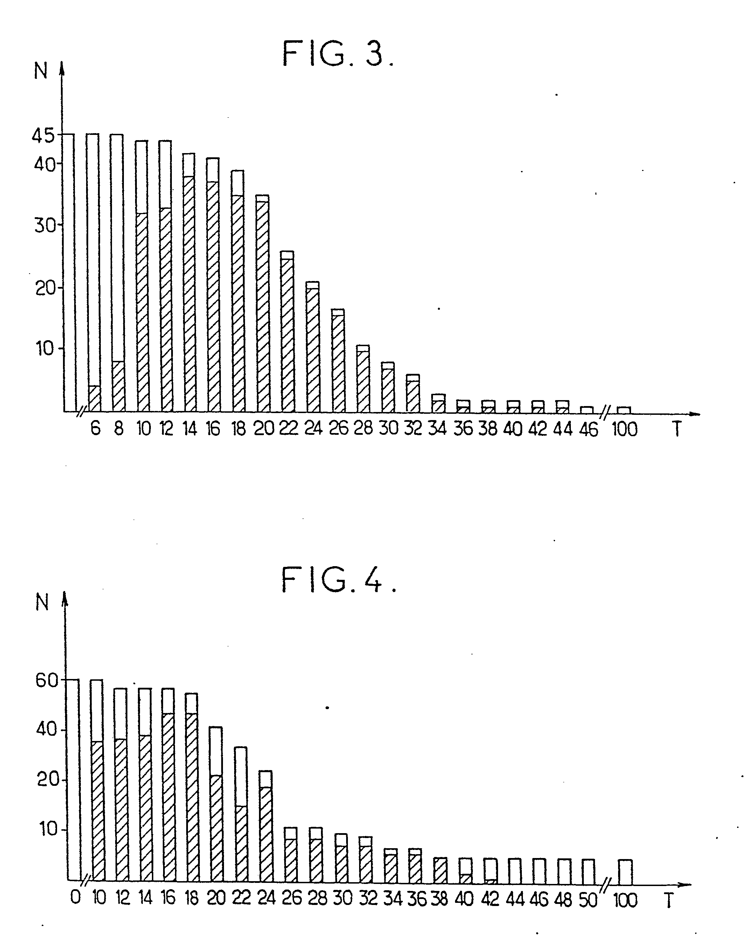

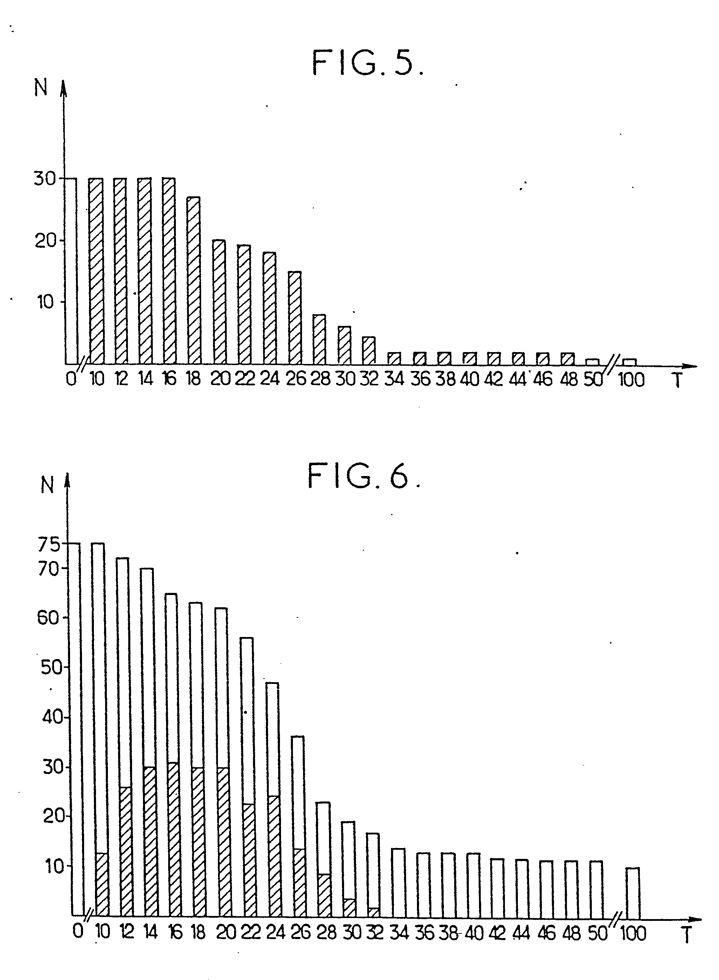

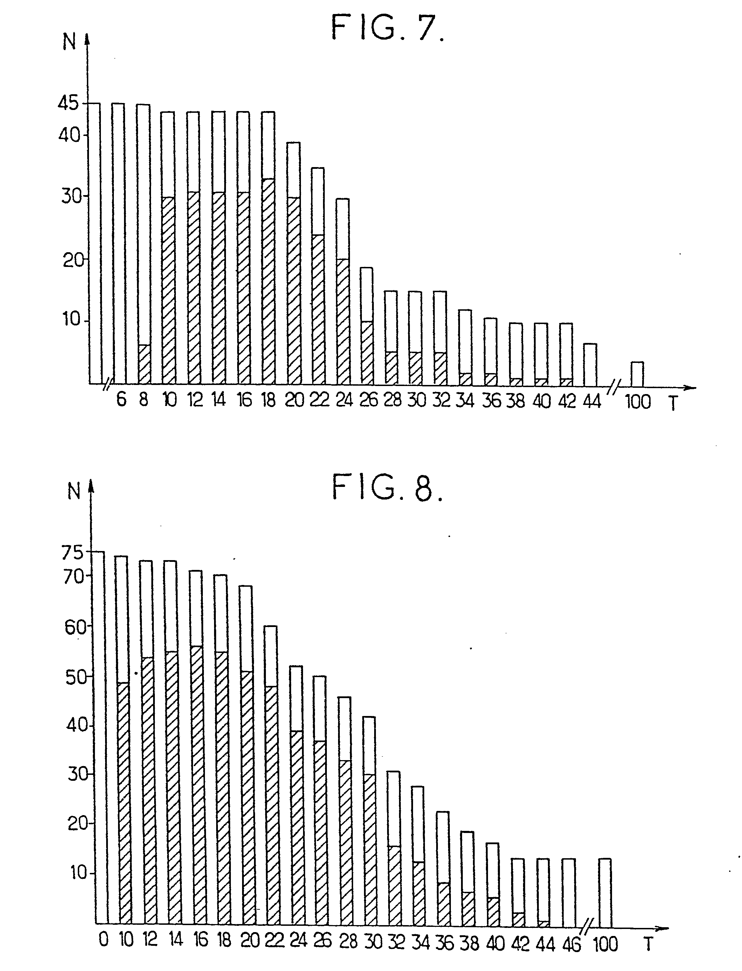

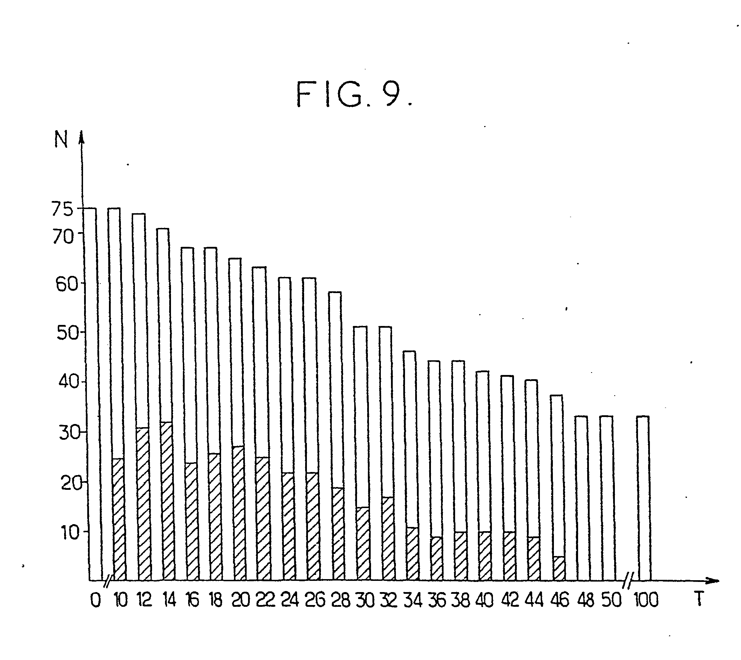

Les résultats de ces expériences apparaissent à l'examen des figures 1 à 9 ci-jointes qui sont des histogrammes correspondant respectivement aux expériences 1 à 9.

Ces histogrammes montrent, en fonction du temps (nombre de jours T), le nombre N d'animaux survivants et, parmi ces animaux survivants, le nombre d'animaux portant des rumeurs décelables.

Ainsi, le nombre d'animaux survivants est porté, pour un jour donné, en ordonnée sous la forme d'une colonne figurant le nombre d'animaux et à l'intérieur de laquelle on matérialise par des hachures le nombre d'animaux porteurs de tumeurs.

De l'examen des histogrammes des figures 1 à 9, il apparaît que:

— dans la première expérience, tous les animaux sont porteurs de tumeurs au 10e jour; au bout de 26 jours, il ne reste que 2 animaux survivants sur 75 au départ, tous deux porteurs de tumeurs et qui meurent au 28e jour;

— dans la deuxième expérience, 43 sur 75 animaux sont porteurs de tumeurs au 10e jour; au bout de 50 jours, il reste 4 animaux dont 2 porteurs de tumeurs. Après une période d'observation de 100 jours, 3 souris survivent définitivement à la greffe cellulaire;

— dans la troisième expérience, 32 sur 45 animaux sont porteurs de temeurs au 10e jour; au bout de 44 jours, il reste 2 animaux dont 1 seul porteur de tumeur. Après une période d'observation de 100 jours, un seul animal survit;

— dans la quatrième expérience, 34 sur 60 animaux sont porteurs de tumeurs au 10e jour et 5 survivent après 100 jours d'observation;

— dans la cinquième expérience, tous les animaux sont porteurs de tumeurs au 10e jour et 1 seul reste survivant après la période d'observation;

— dans la sixième expérience, seul 13 animaux sur 75 sont porteurs de tumeurs au 10e jour et 10 souris survivent après 100 jours d'observation;

— dans la septième expérience, 30 animaux sur 45 sont porteurs de tumeurs au 10e jour; au bout de 44 jours, il reste 7 survivants dont aucun n'est porteur de tumeur et au bout de la période d'observation de 100 jours 4 souris survivent définitivement;

— dans la huitième expérience, 48 sur 75 animaux sont porteurs de tumeur au 10e jour et 14 animaux survivent après 100 jours d'observation;

— dans la neuvième expérience, 25 sur 75 animaux sont porteurs de tumeurs au 10e jour et 33 animaux survivent après une période d'observation de 100 jours.

D'autres expériences montrent que les quantités administrées peuvent être notablement réduites.

En rapport avec l'activité antivirale, ces expériences ont été effectuées en utilisant des solutions de butyrate d'arginine titrant respectivement:

    25 mmoles/litre
    12,5 mmoles/litre
    6,25 mmoles/litre
    3,15 mmoles/litre
    1,5 mmoles/litre

et on a administré pour chaque expérience 0,5 ml de ces solutions par souris de 25 g.

L'interféron a été administré à raison de 20.000 à 25.000 unités internationales par souris par voie intrapéritonéale sous un volume de 0,5 ml et l' immunostimulant, à savoir le corynébacterium parvum, souche Mérieux, a été injecté à l'animal par voie intrapéritonéale à raison de 200 µg/0,1 ml/souris.

L'action antivirale a été étudiée dans les mêmes conditions que plus haut.

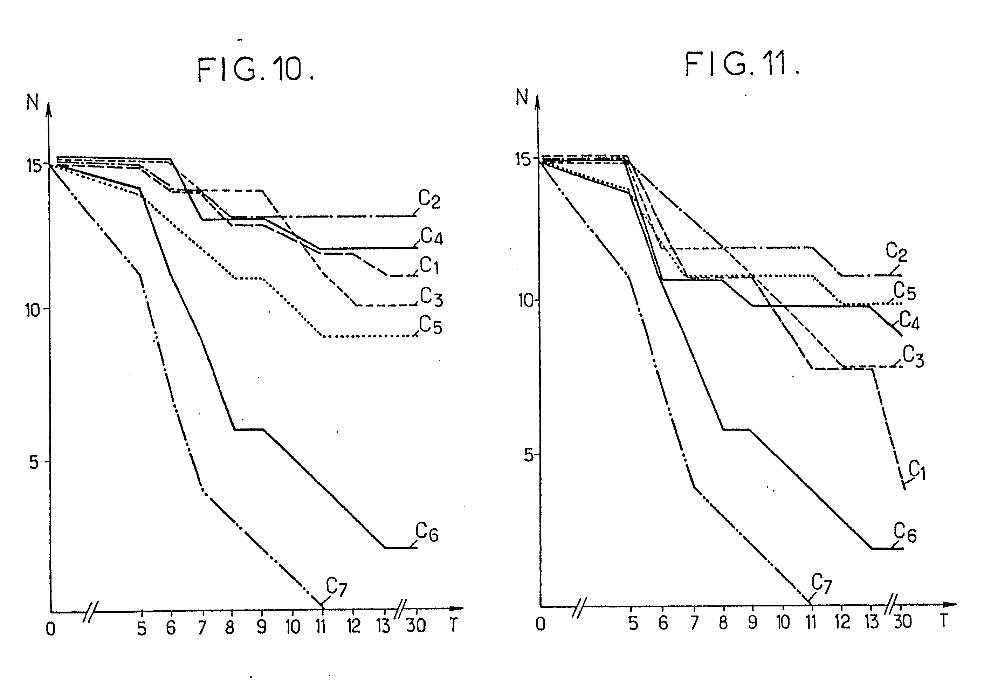

Sur les graphiques (voir figures 10 à 13), on a représenté l'évolution de la survie (nombre de souris survivantes N en fonction du nombre de jours écoulés T) dans des groupes de 15 souris infectées par le virus EMC (100 $DL_{50}$ de virus, 0,5 ml par voie intrapéritonéale) auxquelles a été administré:

— dans le cadre de l'expérience de la figure 10, le butyrate d'arginine aux concnetrations indiquées plus haut, associé à l'interféron et au corynébacterium parvum: les courbes $C_1$ à $C_5$ correspondent respectivement aux concentrations de 25, 12,5, 6,25, 3,15 et 1,5 mmoles/litre; la caurbe $C_6$ correspond au groupe témoin traité par l'interféron seul et la courbe $C_7$ au groupe témoin non traité;

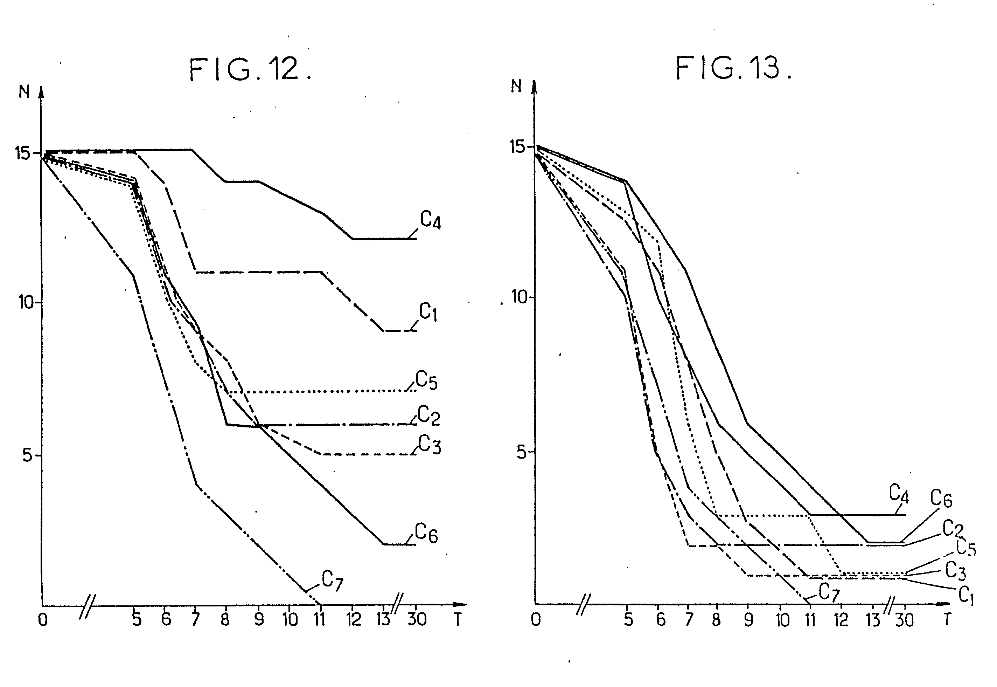

— dans le cadre de l'expérience de la figure 11, le butyrate d'arginine aux concentrations indiquées plus haut, associé au corynébacterium parvum seul: les courbes $C_1$ à $C_5$ correspondent respectivement aux concentrations susdites; les courbes $C_6$ et $C_7$ correspondent aux groupes témoins, comme indiqué pour la figure 10;

— dans le cadre de l'expérience de la figure 12, le butyrate d'arginine aux concentrations indiquées plus haut, associé à l'interféron: les courbes $C_1$ à $C_5$ correspondent respectivement aux susdites concentrations; les courbes $C_6$ et $C_7$ correspondent aux groupes témoins, comme indiqué figure 10.

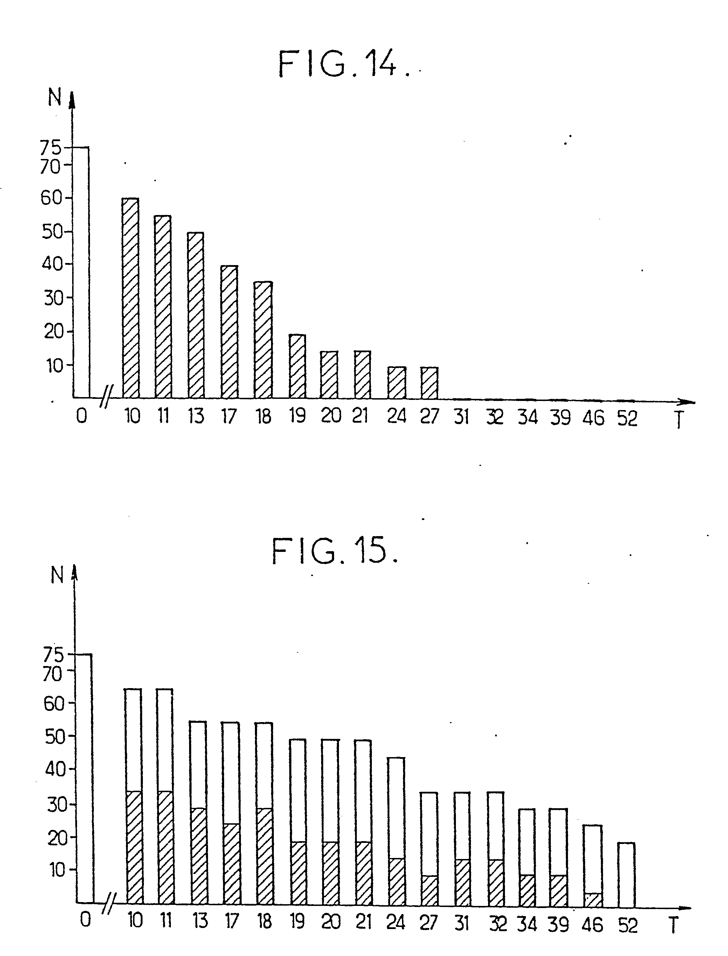

— dans le cadre de l'expérience de la figure 13, le butyrate d'arginine aux concentrations indiquées plus haut, utilisé seul: les courbes $C_1$ à $C_7$ sont identifiées comme plus haut.

De l'examen des graphiques des figures 10 à 13, il résulte que:

— dans le cas de l'association butyrate + immunostirulant + interféron et dans le cas de l'association butyrate + immunostimulant, la dose la plus efficace de butyrate est celle de 0,07 mg/g de poids du corps de souris;

— dans le cas de l'association du butyrate avec l'interféron et dans celui du butyrate seul, la dose la plus efficace de butyrate est de 0,0175 mg/g de poids du corps de souris.

En rapport avec l'activité antitumorale, on a également montré que les quantités administrées pouvaient être abaissées.

Les expériences correspondantes ont été effectuées en utilisant les concentrations et quantités suivantes:

— administration de 0,5 ml par souris de 25 g d'une solution de butyrate d'arginine d'une concentration de 12,5 mmoles par litre, c'est-à-dire 0,07 mg par g du poids du corps de souris;

— administration de 0,5 ml par souris de 25 g d'une solution de butyrate d'arginine d'une concentration de 6,25 mmoles par litre, c'est-à-dire 0,035 mg par g de poids du corps de souris.

Les animaux reçoivent la greffe tumorale ($10^6$ cellules 180 TG de Crocker/0,5 ml/souris par voie intrapéritonéale) au jour 0. Le traitement par le butyrate et l'interféron (20.000 à 25.000 U.I. par voie intrapéritonéale) est commencé au 3ème jour. Ce traitement est poursuivi pendant 3 semaines à raison d'une injection de butyrate suivie au bout de 24 heures par une injection d'interféron, de façon alternative pendant toute la durée du traitement.

Les résultats obtenus sont représentés dans les histogrammes des figures 14 à 19 qui sont établis comme ceux des figures 1 à 9.

L'histogramme de la figure 14 correspond au témoin (souris greffées sans traitement) et montre que toutes les souris sont porteuses de tumeurs au 10e jour et qu'aucune ne survit au-delà de 31 jours.

L'histogramme de la figure 15 (souris greffées traitées par l'interféron seul) montre que l'interféron ralentit légèrement la prise de tumeur, seuls 7 sur 13 animaux étant porteurs de tumeurs au 10e jour; 4 animaux sur 15 survivent après une période d'observation de 52 jours.

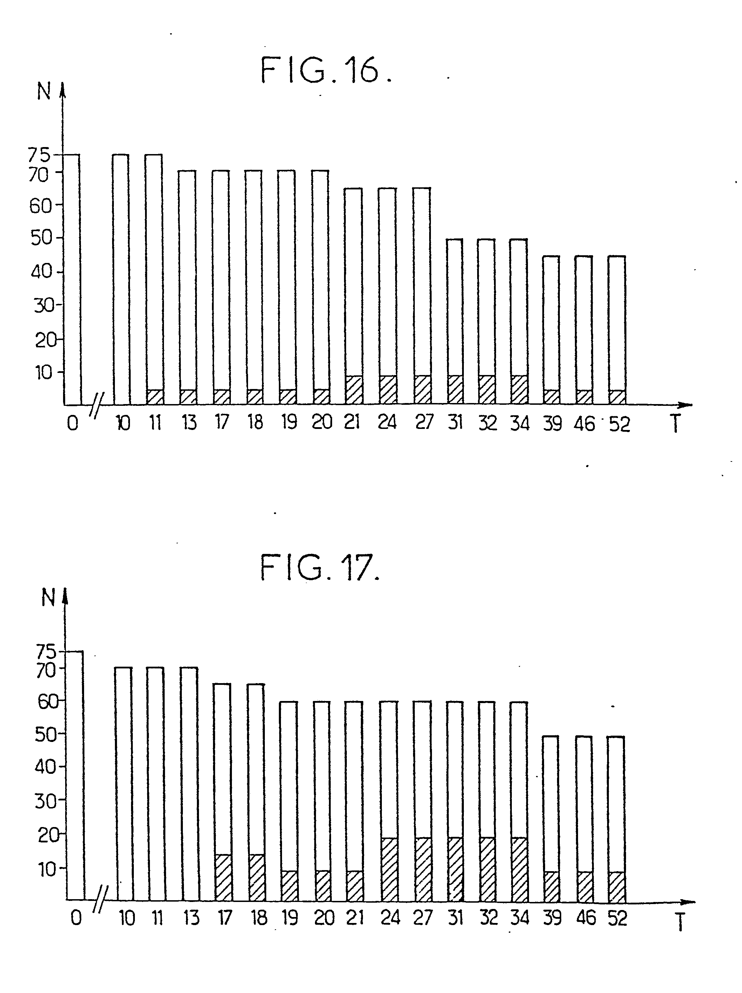

Les histogrammes des figures 16 et 17 (souris greffées et traitées à la dose de 0,07 mg par g de poids du corps de souris de butyrate respectivement seul, figure 16, et associé à l'interféron, figure 17) montrent que l'effet du butyrate seul est considérable puisque à cette dose aucun animal n'est porteur de tumeur au 10e jour. En fin de période d'observation, 9 sur 15 souris survivent dont 1 est porteuse de tumeur. Des résultats analogues sont obtenus lorsque le traitement est combiné à l'interféron (figure 17). Dans ce cas, le retard dans la prise de greffe est important et 9 souris survivent après la période d'observation.

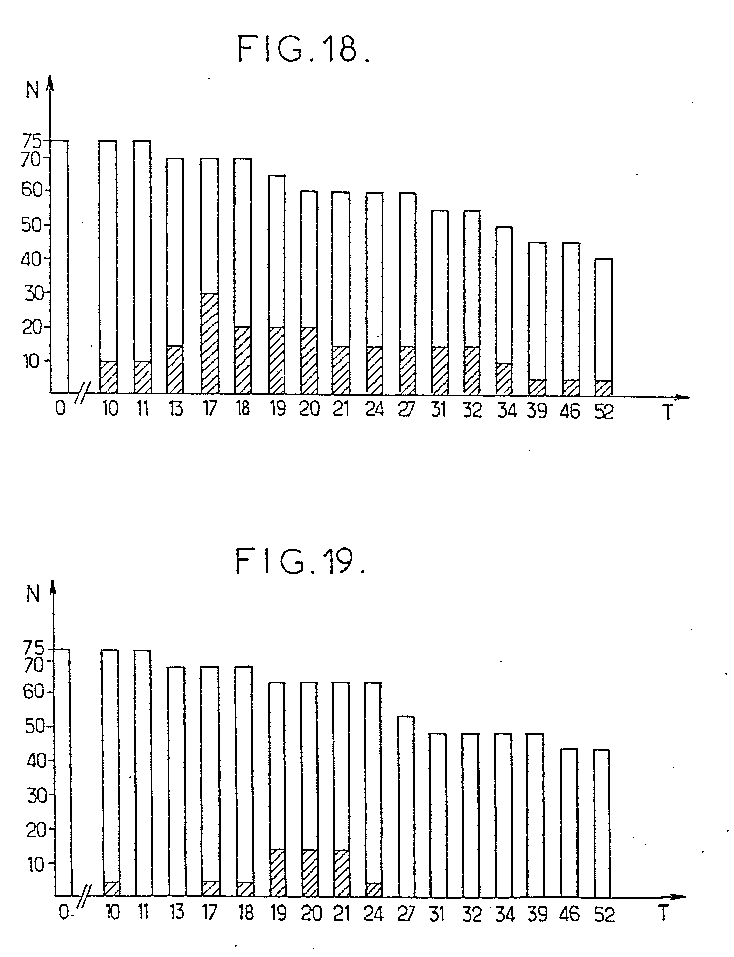

Les histogrammes des figures 18 et 19 correspondent à l'essai du butyrate à la dose de 0,035 mg/g de poids du corps de souris administré seul (figure 18) et associé à l'interféron (figure 19).

Les résultats obtenus avec cette dose de butyrate d'arginine semblent aussi bons que pour la dose double, surtout lorsque l'amino-acide est associé à l'interféron (figure 19). Très peu d'animaux sont alors porteurs de tumeurs et chez certains animaux, celles-ci régressent définitivement. Après une période d'observation de 52 jours, 9 sur 15 souris sont en survie, alors qu'avec le butyrate seul il en reste 8 sur 15 dont 1 encore porteuse de tumeur.

D'une façon générale, il apparaît qu'en association avec l'interféron les doses plus faibles de butyrate semblent posséder une action égale ou supérieure aux doses plus fortes.

L'ensemble des résultats montre que les butyrates d'arginine ou de lysine possèdent un effet antitumoral propre qui est significatif et comparable à celui de l'interféron, au moins dans les conditions expérimentales ici présentées. Il convient d'insister sur le fait que les quantités d'interféron administrées correspondent à la dose optimale. L'administration des butyrates d'aminoacides basiques avec une seule injection de CP augmente considérablement l'effet. Par ailleurs, les butyrates d'arginine ou de lysine agissent en synergie avec l'interféron. L'administration du butyrate d'arginine avec un agent immunostimulant et avec l'interféron aboutit à une augmentation considérable de la protection totale qui se rapproche, dans ce cas, à 50 % de survie définitive.

Les expériences analogues à celles qui viennent d'être décrites, qui ont été effectuées avec le propionate d'arginine (0,252 mg par g de souris) et l'hexanoate d'arginine (0,29 mg par g de souris) à propos des propriétés antivirales de ces produits, ont montré que la survie relevée au moment où, dans la série d'animaux témoins traités uniquement par le virus, la mortalité cesse, est

— de 18 sur 30 animaux dans le cas du propionate d'arginine,

— de 11 sur 30 animaux dans le cas de l'hexanoate d'arginine, et

— de 5 sur 30 animaux témoins qui ont uniquement été traités par le virus.

Comme dans le cas des expériences précédentes, cette survie se trouve considérablement augmentée lorsqu' avec le propionate d'arginine ou l'hexanoate d'arginine, on administre de l'interféron.

Comme il va de soi et comme il résulte d'ailleurs déjà de ce qui précède, l'invention ne se limite nullement à ceux de ses modes d'application et de réalisation qui ont été plus spécialement envisagés; elle en embrasse, au contraire, toutes les variantes.

### Revendications pour les Etats contractants: BE, CH, DE, FR, GB, IT, LI, LU, NL, SE.

1. Médicament antiviral et antitumoral, caractérisé par le fait qu'il comprend, en tant que substance active, au moins l'un des produits de la réaction d'un acide carboxylique du groupe comprenant les acides butyrique, propionique et hexanoïque sur un amino-acide basique du groupe constitué par l'arginine et la lysine.

2. Médicament antitumoral caractérisé par le fait qu'il comprend, en tant que substance active, au moins l'un des produits de la réaction d'un acide carboxylique du groupe comprenant les acides butyrique, propionique et hexanoïque sur un amino-acide basique du groupe constitué par l'arginine et la lysine ainsi que de l'interféron et/ou un agent immunostimulant.

3. Médicament selon l'une des revendications 1 et 2, caractérisé par le fait que la substance active comprend du butyrate d'arginine et/ou de lysine.

4. Médicament selon l'une des revendications 1 et 2, caractérisé par le fait que la substance active comprend de l'hexanoate d'arginine.

5. Médicament selon la revendication 2, caractérisé par le fait que l'agent immunostimulant est choisi dans le groupe comprenant le corynébacterium parvum, l'isoprinosine, l'extrait de staphylocoques, la cimétidine et le lévamisole.

6. Médicament selon l'une des revendications 1 à 5, caractérisé par le fait qu'il comprend un excipient non toxique et pharmacologiquement acceptable et qu'il est administré par les voies parentérale, intraveineuse ou orale.

### Revendications pour l'Etat contractant: AT

1. Procédé de fabrication de médicament antiviral et antitumoral, caractérisé en ce qu'on fait réagir au moins un acide carboxylique de groupe comprenant les acides butyrique, propionique et hexanoique sur au moins un amino-acide basique du groupe constitué par l'arginine et la lysine.

2. Procédé de fabrication d'un médicament antitumoral caractérisé en ce qu'on fait réagir au moins un acide carboxylique du groupe comprenant les acides butyrique, propionique et hexanoique sur au moins un amino-acide basique du groupe constitué par l'arginine et la lysine, et on ajoute de l'interféron et/ou un agent immunostimulant au produit de la réaction.

3. Procédé selon l'une des revendications 1 et 2, caractérisé en ce que la réaction est effectuée pour obtenir, en tant que substance active, du butyrate d'arginine et/ou de lysine.

4. Procédé selon l'une des revendications 1 et 2, caractérisé en ce que la réaction est effectuée pour obtenir, en tant que substance active, de l'hexanoate d'arginine.

5. Procédé selon la revendication 2, caractérisé en ce que l'agent immunostimulant ajouté est choisi dans le groupe comprenant le corynébacterium parvum, l'isoprinosine, l'extrait de staphylocoques, la cimétidine et le lévamisole.

6. Procédé selon l'une des revendications 1 à 5, caractérisé en ce qu'on ajoute un excipient non toxique et pharmacologiquement acceptable, en vue d'une administration par voie parentérale, intraveineuse ou orale.

**Patentansprüche**

för die Vertiagsstaaten: BE, CH, DE, FR, GB, IT, LI, LU, NL, SE

1. Antivirales und antitumorales Arzneimittel, dadurch gekennzeichnet, dass es als Wirkstoff zumindest eines der Produkte der Umsetzung einer Carbonsäure der Gruppe umfassend Buttersäure, Propionsäure und Hexansäure mit einer basischen Aminosäure der Gruppe bestehend aus Arginin und Lysin, enthält.

2. Antitumorales Arzneimittel, dadurch gekennzeichnet, dass es als Wirksubstanz zumindest eines der Produkte der Umsetzung einer Carbonsäure der Gruppe umfassend Buttersäure, Propionsäure und Hexansäure mit einer basischen Aminosäure der Gruppe bestehend aus Arginin und Lysin, sowie Interferon und/oder ein immunostimulierendes Mittel enthält.

3. Arzneimittel nach einem der Ansrpüche 1 und 2, dadurch gekennzeichnet, dass der Wirkstoff Argininbutyrat und/oder Lysin enthält.

4. Arzneimittel nach einem der Ansprüche 1 und 2, dadurch gekennzeichnet, dass der Wirkstoff Argininhexanoat enthält.

5. Arzneimittel nach Anspruch 2, dadurch gekennzeichnet, dass das immunostimulierende Mittel aus der Gruppe umfassend Corynebacterium parvum, Isoprinosin, Staphylokokkenextrakt, Cimetidin und Levamisol gewählt ist.

6. Arzneimittel nach einem der Ansprüche 1 bis 5, dadurch gekennzeichnet, dass es einen nicht-toxischen und pharmakologisch verträglichen Trägerstoff enthält, und parenteral, intravenös oder per os verabreicht wird.

**Patentansprüche**

für den Vertragstaat: AT

1. Verfahren zur Herstellung eines antiviralen und antitumoralen Arzneimittels, dadurch gekennzeichnet, dass zumindest eine Carbonsäure der Gruppe umfassend Buttersäure, Propionsäure und Hexansäure mit zumindest einer basischen Aminosäure der Gruppe bestehend aus Arginin und Lysin umgesetzt wird.

2. Verfahreb zur Herstellung eines antitumoralen Arzneimittels, dadurch gekennzeichnet, dass man zumindest eine Carbonsäure der Gruppe umfassend Buttersäure, Propionsäure und Hexansäure mit zumindest einer basischen Aminosäure der Gruppe bestehend aus Arginin und Lysin umsetzt, und dass man dem Reaktionsprodukt Interferon und/oder ein immunostimulierendes Mittel zusetzt.

3. Verfahren entsprechend einer der Ansprüche 1 und 2, dadurch gekennzeichnet, dass die Umsetzung zur Erhaltung, als Wirksubstanz, von Argininbutyrat und/oder Lysinbutyrat durchgeführt wird.

4. Verfahren nach einem der Ansprüche 1 und 2, dadurch gekennzeichnet, dass die Umsetzung zur Erhaltung, als Wirkstoff, von Argininhexanoat durchgeführt wird.

5. Verfahren nach Anspruch 2, dadurch gekennzeichnet, dass das zugefügte immunostimulierende Mittel aus der Gruppe umfassend Corynebacterium parvum, Isoprinosin, Staphylokokkenextrakt, Cimetidin und Levamisol gewählt wird.

6. Verfahren nach einem der Ansprüche 1 bis 5, dadurch gekennzeichnet, dass ein nicht-toxischer· . und pharmakologisch verträglicher Wirkstoff zugefügt wird, zur paranteralen, intravenösen oder oralen Verabreichung.

**Claims**

for the contracting States: BE, CH, DE, FR, GB, IT, LI, LU, NL, SE

1. Antiviral and antitumoral drug, characterized by the fact that it contains as active substance at least one of the products of the reaction of a carboxylic acid of the groups comprising butyric, propionic and hexanoic acid with an amino-acid of the group consisting of arginine and lysine.

2. Antitumoral drug, characterized by the fact that it contains as active substance at least one of the products of the reaction of a carboxylic acid of the group comprising butyric, propionic and hexanoic acid with an amino-acid of the group consisting of arginine and lysine as well as interferon and/or an immunostimulant agent.

3. Drug according to one of claims 1 and 2, characterized by the fact that the active substance comprises arginine butyrate and/or lysine butyrate.

4. Drug according to one of claims 1 and 2, characterized by the fact that the active substance comprises arginine hexanoate.

5. Drug according to claim 2, characterized by the fact that the immunostimulant agent is selected from the group comprising corynebacterium parvum, isoprinosine, staphylococci extract, cimetidine and levamisole.

6. Drug according to one of claims 1 to 5, characterized by the fact that it comprises a non-toxic and pharmacological acceptable excipient and that it is administered parenterally, intraveneously, or orally,

## Claims

for the contiacting State: AT

1. Process for the manufacture of an antiviral and antitumoral drug, characterized by the fact that at least one carboxylic acid of the groups comprising butyric, propionic and hexanoic acid is reacted with at least one basic amino-acid of the group consisting of arginine and lysine.

2. Process for the manufacture of an antitumoral drug, characterized by the fact that at least one carboxylic acid of the group comprising butyric, propionic and hexanoic acids is reacted with at least one basic amino-acid of the group consisting of arginine and lysine and that interferon and/or an immunostimulant agent is added to the product of the reaction.

3. Process according to one of claims 1 and 2, characterized by the fact that the reaction is effected in order to obtain, as active substance, arginine butyrate and/or lysine butyrate.

4. Process according to one of claims 1 and 2, characterized by the fact that the reaction is effected in order to obtain, as active substance, arginine hexanoate.

5. Process according to claim 2, characterized by the fact that the immunostimulant agent which is added is selected from the group comprising a corynebacterium parvum, isoprinosine, staphylococci extract, cimetidine an levamisole.

6. Process according to one of claims 1 to 5, characterized by the fact that a non-toxic and pharmacologically acceptable excipient is added with the aim of a parenterally, intraveneously or orally administration.

# FIG.1.

# FIG.2.

# FIG. 3.

# FIG. 4.

# FIG. 5.

# FIG. 6.

# FIG. 7.

# FIG. 8.

# FIG. 9.

FIG. 11.

FIG. 10.

FIG.13.

FIG.12.

# FIG.14.

# FIG.15.

# FIG.16.

# FIG.17.

# FIG.18.

# FIG.19.